# EUROPÄISCHE PATENTANMELDUNG

(11) **EP 2 703 044 A1**
(43) Veröffentlichungstag der Anmeldung: **05.03.2014**
(21) Anmeldenummer: 11864224.8
(22) Anmeldetag: 29.04.2011
(51) Int. Cl.: A61N 5/00, A61N 5/067

(54) **FELDEFFEKTHARMONISIERER FÜR BIOSYSTEME UND STOFFE**

(30) Priorität: 25.04.2011 RU 2011115987
(71) Anmelder: Vorsunov, Gennady Vasilievich, Moskovskaya obl. 142203 (RU)
(72) Erfinder: Vorsunov, Gennady Vasilievich, Moskovskaya obl. 142203 (RU)
(74) Vertreter: Jeck, Anton
(86) Internationale Anmeldenummer: PCT/RU2011/000292
(87) Internationale Veröffentlichungsnummer: WO 2012/148302

(57) **Zusammenfassung**

Die Erfindung gehört zum Gebiet der Biotechnologien und der Technologien für Gesundheitswesen und Umwelt. Das technische Ergebnis bei der Verwendung der Erfindung ist die Bildung eines biologisch aktiven Zustands für die Übertragung der Organismen der Menschen, Tiere und anderer Biosystemen aus dem disharmonischen in den harmonischen und gesunden Zustand. Der Feldharmonisator für Biosysteme gilt als Quelle des Felds. Die Einrichtung besteht aus einer Disk mit eingespeicherten biologisch aktiven Filmen (BAF), einem Reflektor aus Spiegeln, parabolisch oder flach oder aus Alufolie, oder einer Disk mit der auf dieser befestigten Platten mit einem Film, der von der Disk nach der Aufnahme der BAF abgenommen ist oder einem Reflektor und einer oder mehreren auf diesem fixierten Packungen der Platten mit Aufnahmen der BAF, die auf der Zellenkonstruktion fixiert sind. Dabei können eine große Menge von Zellenformen verwendet werden, wobei die äußere Kontur der Zellen in Form eines Zylinders oder eines mehrflächigen Prismas beschränkt ist. Innerhalb der Zellen liegen Zylinder und Prismen kleinerer Größe, die sich miteinander kreuzen oder nicht kreuzen, unter Erhaltung der Symmetrie in Bezug auf die Achse der Zellen, wobei alle Elemente eine lichtabstrahlende Abdeckung aufweisen. Auf der zellenförmigen Konstruktion können Konusse oder Pyramiden oder Linsen aus Glas, Kristall oder natürliche bzw. künstliche Mineralien befestigt werden. Die Einrichtung kann mindestens aus einem Bündel optischer Fasern, einem Laser und einem Projektor bestehen.

## Beschreibung

Die Erfindung gehört zum Gebiet der Biotechnologie, Technologie für Gesundheitswesen und Umwelt und ist für ein biologisch aktives Feld mit bedingter wirkungsvoller Leistung zur Übertragung der Organismen der Menschen, Tiere und anderer Biosysteme aus dem disharmonischen und kranken in den harmonischen und gesunden Zustand zur Steigerung der Lebensruhespannung, Verzögerung der Alterungsvorgänge, Verlängerung der aktiven Langlebigkeit, Lösung der Umweltprobleme, Verbesserung der Gebrauchseigenschaften unterschiedlicher Substanzen und Sachen vorgesehen, die von einem Menschen im Verlauf des Lebens benutzt werden. Es ist auch die Verwendung in einem breiteren Aufgabenkreis möglich.

Alle im Folgenden beschriebenen Einrichtungen gelten als Quelle von biologisch aktiven Feldern, die den Zustand von Biosystemen, Wasser und anderen Substanzen, die sich in flüssiger und halbflüssiger Form befinden, harmonisieren. Als Grundlage dieser Einrichtungen gelten die biologisch aktiven Filme (BAF) von Gennadij Worsunow.

Die biologisch aktiven Filme (BAF) von G. Worsunow schaffen bei ihrer Übertragung auf beliebige Bildschirme von Fernsehern, Computern usw. Felder, die auf die biologischen Felder von lebendigen Organismen einwirken und diese aus dem disharmonischen Zustand, der dem kranken Organismus entspricht, in den gesunden, harmonischen Zustand übertragen. Die Harmonisierung des Biofelds regt in diesem unterschiedliche biophysische, biochemische und andere Prozesse an, die viele Gesundheitsprobleme zu lösen erlauben, darunter auch solche, die schwer zu lösen oder mit der klassischen Medizin nicht zu lösen sind. Autorenrechte auf diese Filme und das Projekt für ihre Anwendung und ihre Beschreibung sind registriert: G.Worsunow, Copyright, THREAD ID 1-70 HVHU, THREAD ID 1-70JF6Y, 06/15/2010.

Eine der Ideen, die bei der Herstellung dieser Filme verwendet wurde, hat G.W. Worsunow in Deutschland patentieren lassen, s. Gebrauchsmuster 21 2008 000 034.2 vom 04.03 2010.

Die in diesem Patent beschriebene Einrichtung gilt als Prototyp für die Ausarbeitung der vorliegenden Erfindung. Der Mangel dieser bekannten Einrichtung besteht in der großen Abmessung, so dass ihre Verwendungsmöglichkeiten beschränkt sind.

Alle Einrichtungen sind an Freiwilligen ausprobiert worden, die chronische oder einmalige Gesundheitsprobleme hatten. In den meisten Fällen waren gute Ergebnisse in folgenden Richtungen erzielt worden: bei chronische Schmerzen in den Gelenken, im Bereich des Herzens und des Kopfs, Heilung nach schweren Verletzungen des Gesichts und des Kopfs, Erholung von Kleinstkindern und Kindern höheren Alters nach Geburtstraumatas, eine Verbesserung der Rede und Koordination der Bewegungen bei einem Mädchen mit Zerebralparese, Wiederherstellung der Logik und des Gedächtnisses bei älteren Leuten, Beseitigung der Meteo-Abhängigkeit (Wetterabhängigkeit) und anderes mehr.

Darüber hinaus haben die Wissenschaftler im Labor der OOO-KTI (Stadt Sankt Peterburg, Russland, www.kti.spb.ru, e-mail:info@kti.spb.ru), im Auftrag von G.W. Worsunow mit Hilfe einer GDV-Kamera im März 2011 experimental nachgewiesen, dass das Wasser auf Felder reagiert, die von den Harmonisatoren der Biosysteme von G.W. Worsunow ausgestrahlt werden. Auch mit Hilfe der GDV-Kamera von Korotkow registriert G.W. Worsunow die Reaktionen von Menschenorganismen, Zahnpasta und Cremes auf diese Felder. Die Reaktionen von kranken Menschen auf die Felder der BAF, die von BAF und der Einrichtung gemäß der Erfindung geschaffen werden, zeigt Aufnahmen von kranken Stellen im Infrarotgebiet im Verlauf der Einwirkung der Felder auf den Organismus. Eine Infrarot-Kamera mit einer Empfindlichkeit von 0,05° C registriert die Verminderung der Temperatur auf den Problemstellen auf 0,5° und mehr nach 15 - 30 Minuten der Einwirkung der Einrichtung gemäß der Erfindung und der BAF.

Die Felder der Einrichtung gemäß der Erfindung registriert eine Messeinrichtung für geophysikalische Anomalien (IGA1) von J.P. Krawchenko (www.iga1.ru). Im Artikel von G.I. Schipow "Über die experimentelle Vermessung der Wellenfunktion der Quantenmechanik" sind auf Seite 22 Experimente für die Vermessung der Ausstrahlungen der Torsionsfelder mit Hilfe dieses Geräts beschrieben (Artikel auf http://.spinor.kiev.ua).

Der Doktor für Biologie P. Gariaew (http:// p-gariaev.narod.ru/wave_gen_intro.htm) betrachtet das Genom eines Menschen als Quantencomputer, der eine spintorsionale Aufnahme, Informationsspeicherung und die Lieferung der Information an die Zellen erfüllt. Er hat ein wirksames Modell des Biocomputers geschaffen, in dem die Information abgelesen und mit Hilfe eines Laserstrahls übertragen wird. Die in diesem Modell erhaltene Information wird als Programmierungsmittel der Organismuszellen für die Verjüngung, Heilung usw. verwendet. Die Eigenschaft des Lasers, die Information zu erfassen und diese zu übertragen, wurde von G.W. Worsunow in den Einrichtungen Nr. 8 und Nr. 9 verwendet.

Die Änderungen der Feldflächen, registriert mit der GDV-Kamera, für Wasser, Zahnpasta, Creme, Menschenorganismus unter der Einwirkung der Felder der Einrichtung gemäß der Erfindung und die Verminderung der Körpertemperatur in den anomalen Bereichen des Organismus weist wahrheitsgetreu selbst die Tatsache des Vorhandenseins der Felder und deren Arbeitsfähigkeit nach. Die Verbesserung der Gesundheit bei den Benutzern der Einrichtungen und der BAF beweisen die positive Einwirkung auf die Organismen eines Menschen (auch auf die der Hunde und Katzen).

Als technisches Ergebnis der Erfindung gilt die Schaffung von Technologien für das Gesundheitswesen, die Umwelt und Biotechnologien, die in sich die Wirksamkeit mit niedrigen Kosten und die Einfachheit und Behaglichkeit der Anwendung mit absoluter Sicherheit vereinigen.

Das angegebene technische Ergebnis wird dadurch erzielt, dass der Feldharmonisator von Biosystemen und Substanzen einen Diskharmonisator zur Schaffung von auf die Lebensorganismen positiv einwirkenden Feldern, einschließlich des Menschenorganismus, und zur Einwirkung auf die Substanzen mit einer Disk, auf der 23.5 und mehr Gbyte biologisch aktiver Filme, kurz BAF, die von G. Worsunow aufgenommen waren, oder mit einer Disk und auf dieser fixierten Platten aus Filmen enthält, die aus den Disks nach der Aufnahme der BAF auf diesen abgenommen waren, oder aus einer bestimmten Anzahl solcher Platten, die in die Packungen hereingelegt waren; oder aus anderen Informationsträgern, die die Information mit der Qualität und in der Anzahl ausreichend für die Anregung der positiven Änderungen in Biosystemen einspeichern, liegend auf beliebiger Oberfläche mit einer lichtreflektierenden Abdeckung aus Alufolie, aus Spiegeln oder aus poliertem Metall usw. zum Reflektieren des Felds zum Biosystem.

Dabei schließt der Feldharmonisator der Biosysteme und der Substanzen Konstruktionen ein, die aus Zellen bestehen, die viele Formen aufweisen können, doch deren gemeinsame Merkmale die Form der Außenkontur der Zellen in Form eines regelmäßigen vielflächigen Prismas oder eines Zylinders und die symmetrische Lage der Zellen in Bezug auf deren Achse sind. Dabei sind die Zylinder und Prismen von geringerer Größe, die sich untereinander kreuzen oder nicht kreuzen, eine reflektierende Abdeckung oder polierte Oberflächen aufweisen und auf der Außenseite in Bezug auf das Biosystem angeordnet sind. Ferner liegen die Zellenseiten der Einrichtungen wie in der Einrichtungsvariante 1, Linsen sind innerhalb der Zellen angeordnet, und die Konusse und Pyramiden bestehen aus Glas, Kristall oder Mineralien und sind mit ihren scharfen Spitzen zum Biosystem gerichtet. Schließlich ist dabei ein für die ganze Einrichtung gemeinsamer oder ein getrennter Feldreflektor für jede Zelle aus Aluminiumfolie oder ein die Felder des Biosystems reflektierender Spiegel vorgesehen.

Auf der Oberfläche der Einrichtung werden ein oder mehrere Bündel optischer Fasern mit einem der beiden Enden oder mit beiden Enden fixiert, wonach die Harmonisatoren gemäß Anspruch 1 oder 2 oder ihre Kombination zusammen mit den auf ihnen fixierten Enden der optischen Faser auf der Oberfläche mit der lichtreflektierenden Abdeckung fixiert oder in Alufolie eingewickelt oder in einer Kiste untergebracht werden, deren Innenflächen aus Spiegeln hergestellt werden, oder eine andere lichtreflektierende Abdeckung besitzen. Die optische Faser liegt dabei über den Grenzen der Kiste bzw. einer Folie oder befindet sich im freien Zustand und wird für die Errichtung des Felds zu einem Biosystem oder der Substanz verwendet oder verzweigt sich auf einige Bündel und befördert das Feld zu einigen Biosystemen und den Substanzen gleichzeitig, oder die optische Faser wird auf der Oberfläche des Spiegels oder auf einer anderen Oberfläche mit der lichtreflektierenden Abdeckung verteilt und fixiert, oder sie wird gleichmäßig auf das Gehäuse mit späterer Fixierung auf der Schicht der optischen Reflektorfaser aus den Spiegeln oder aus der Alufolie eingewickelt, die das Feld in den inneren Raum reflektiert, der mit der Faser beschränkt ist und in dem das die Feldeinwirkung benötigende Biosystem liegt.

Einrichtungen gemäß den Ansprüchen 1, 2 oder 3 werden in diversen Varianten, die den zu lösenden Aufgaben und technischen Möglichkeiten ihrer Befestigung und Betriebsweise entsprechen, auf der Oberfläche von drehbaren Teilen oder von deren Innenhohlräumen, beispielsweise von Flügeln, Lüfterrotoren, Luft- und Wassergeneratoren, Schiffspropellern, Fahrzeugrädern und Reifen von Autos und anderen Transportmitteln, befestigt, wobei die rotierenden Elemente dieser Teile besonders für die Erzeugung und Abstrahlung in den Raum von biologisch aktiven Feldern ausgebildet sind, die die biologischen Systeme und Substanzen in positiver Weise beeinflussen.

Der Feldharmonisator für Biosysteme und Substanzen kann in einem weiten Bereich und in allen Formen und Größen angewendet werden: angefangen bei einer Packung zur Aufbewahrung von Parfümerzeugnissen, Wasser, Nahrungsmitteln, Alkohol, Medikamenten bis hin zu Kammern und Räumlichkeiten zur Erfüllung der Anforderungen an die Gesundheit und Biotechnologie. Er wird durch jeden Raum dargestellt, der an Oberflächen mit lichtreflektierenden Oberflächen gebunden ist und dessen Innenseite durch die Einrichtungen gemäß Anspruch 1, 2, 3 oder 4 in Kombinationen gebildet ist, die den mittels dieser Einrichtungen zu lösenden Aufgaben entsprechen.

Im Feldharmonisator der Biosysteme und Substanzen werden die Bündelenden der optischen Fasern in der Einrichtung gemäß Anspruch 5 fixiert.

Auf den Eingang in die optische Faser wird durch das Objektiv des Projektors die Darstellung BAF übertragen oder der Laserstrahl oder einige Laserstrahlen, moduliert mit der Darstellung BAF oder nicht moduliert, vom Laser (von den Lasern) liegend innerhalb der Einrichtung gemäß Anspruch 5 oder 3. Der Strahl des Lasers oder der Laser des Feldharmonisators der Biosysteme und der in der Kiste liegenden Substanzen wird durch eine Öffnung an der Wand der Kammer zum Biosystem gerichtet, das die Einwirkung benötigt.

### Einrichtungsvariante 1

Der Feldharmonisator für Biosysteme besteht aus einer Disk mit einer Aufnahme der BAF von G.W. Worsunow in der Größe von 23.5 und mehr Gbyte, aus einem Reflektor aus Aluminiumfolie oder aus einem flachen bzw. parabolischen Spiegel, der an die der Arbeitsseite gegenüberliegenden Seite (der Etikettseite) der Disk angeklebt und an der Arbeitsfläche der Diskplatten der Filme befestigt ist, die von derjenigen Disk abgenommen sind, auf der vor dem Entfernen der Platten die BAF aufgenommen worden waren.

Wie die Experimente von G.W. Worsunow zeigen, ist die Wirksamkeit der Einwirkung dieser Einrichtungen direkt proportional der Anzahl der Informationen, die in der Einrichtung vorhanden sind, und der Wirksamkeit der BAF selbst.

Für die Wirkungserhaltung bei der Lösung der Gesundheitsprobleme ist es ausreichend, diese Einrichtung mit dem Reflektor nach außen auf dem Körper eines Menschen im anomalen Bereich unterzubringen. Bei einer bequemeren Variante werden die Platten mit der Aufnahme auf den Grund aus dünnem, flexiblem Grundstoff vorbedeckt mit Alufolie. In schwierigeren Situationen werden Packungen aus einer großen Plattenmenge verwendet. Man kann diese Einrichtungen in beliebigen Abmessungen herstellen, wobei man sie auf der reflektierenden Oberfläche mit der notwendigen Abmessung nebeneinander befestigt, und zwar die Packungen aus den Platten zu je 5 - 10 und mehr Stück (240 Gbyte Information BAF). Bei der ständigen tagtäglichen Verwendung solcher Einrichtungen verschwinden die Gesundheitsprobleme in einigen Tagen oder Monaten vollständig oder teilweise. Während des Schlafens werden die Einrichtungen unter den Bezug des Kissens, des Lakens usw. verlegt, und am Tage werden sie auf dem Körper in den Bereichen, in denen Probleme bestehen, untergebracht. Besonders wirkungsvoll ist die Verwendung bei Kindern. Bei unkomplizierten Problemen, wie bei Verschlimmerung der Gastritis, Monatsschmerzen bei Frauen, Unbehagen nach Konsum von Alkohol, spontan entstandenen Kopfschmerzen oder Schmerzen im Bereich der Leber, der Nieren, des Herzens usw., ist es ausreichend, die Einrichtung im entsprechenden Bereich 30 - 60 Minuten zu halten, und Schmerzen und Unbehagen nehmen ab. Dagegen kann es bei chronischen Schmerzen Monate dauern. Es wurden mit Hilfe dieser Einrichtung auch Probleme bei Tieren gelöst.

Mit dem Erscheinen der Blue-ray-Disks, die eine Information von 320 Gb aufnehmen können, und der holographischen Disks mit mehr als 1.6 Tb (solche Disks sind in Japan hergestellt worden) wird der Prozess der Schaffung leistungsstarker Einrichtungen viel einfacher, und die Einrichtungen selbst werden kompakter.

Der Austritt der Emission aus der Oberfläche der Disks und Platten, die aus Filmen besteht, die auf der Disk nach der Aufzeichnung der BAF gespeichert wurden, ist für die Tatsache verantwortlich, dass während der Aufzeichnung der bioaktiven Filme (BAF) von G.W. Worsunow auf der Disk in der Größe von Nanometern in dem als Informationsträger dienenden Film ein Laser eine harmonische, voluminöse, spiralähnliche Struktur einbrennt, die die BAF-Information bildet. Das von dieser Struktur ausgesandte Torsionsfeld trägt die BAF-Information und ruft im Organismus verschiedene biophysische, biochemische und andere Prozess hervor, die eine gute Gesundheit sichern. Aufgrund geringer Abstände der Spirale im Blue-Ray-Aufnahmeformat nähert sich die Spirale einem System aus konzentrischen Kreisen, und gemäß dem russischen Wissenschaftler G.I. Shipow (www.shipov.com), (www.fizvakum.narod.ru), Kapitel 4 und dem ukrainischen Wissenschaftler A.R. Pavlenko in "Computer, Zelle...und Gesundheit?", 2007, S. 180, Ukraine, Kiew, Osnova, ISBN 978-699-266-9, besitzt dieses System einen Umfang, der ein Maximum an Torsionskontrast aufweist (http://www.spinor.kiev.ua).

Die Abstrahlung des Felds von der Oberfläche der Filme nach der Aufnahme der BAF auf diese wird "Wirkung der Hohlstrukturen" genannt; diese Bezeichnung ist vom russischen Entomologen W.S. Grebennikow (http://www.incognita.ru/hronik/pla-net/p 020.htm) formuliert worden. Die für alle bekannte Wirkung der Hohlstrukturen sind Felder, die Rahmen mit Honigwaben ohne Honig bilden, die nebeneinander aufgestellt sind.

Entsprechend der Theorie der Torsionsfelder tragen sie keine Energie, dafür aber die Information. Für den Schutz vor schädlichen Torsionsfeldern, ausgestrahlt von Computern, Telefonen usw., hat A. P. Pavlenko die Einrichtung Forpost 1 entwickelt, s. United States Patent 6,548,752, Pavlenko et al, April 15, 2003 (www.spinor.kiev.ua).

Den Mechanismus der Einwirkung der Information auf lebendige Organismen hat Professor Masaru Emoto aus Japan (www.masaru-emoto.net, www.hado.net) formuliert. Er hat mit unzähligen Experimenten nachgewiesen, dass das Wasser unter Einwirkung einer Information seine Struktur adäquat dem Inhalt der Information ändert. Und da der Organismus eines Menschen und aller Biosysteme hauptsächlich aus Wasser besteht, geht der Organismus unter Einwirkung der äußeren Information mit einem positiven Charakter aus dem kranken Zustand in den gesunden Zustand über. Mit den experimentalen Untersuchungen der Wassereinwirkung auf verschiedene äußere Faktoren befasst sich der Doktor für technische Wissenschaften K.G. Korotkow (www.korotkov.org). Er hat experimental nachgewiesen, dass die Gesinnung eines Menschen (d. h. das Feld bewusst erzeugt und ausgestrahlt) auf den Zustand des Wassers einwirkt. Auf den Disks in der Einrichtung gemäß Anspruch 1 wurden biologisch aktive Filme aufgenommen, das heißt, die Information, die auf die Unterstützung des Zustands der lebendigen Organismen im harmonischen, gesunden Zustand gerichtet ist. Deswegen ändert das Wasser unter Einwirkung dieser Information seine Struktur adäquat dem Inhalt der Information, und es erfolgt ein Übergang des Organismus aus dem kranken in den gesunden Zustand.

Der Vorteil dieser Einrichtung besteht in ihrer Einfachheit bei der Herstellung und Verwendung, in der Möglichkeit ihrer Ausführung in der Mehrzahl der Varianten, wie nach den Abmessungen als auch nach der bedingten wirkungsvollen Leistung auf Kosten der Variabilität der Informationsmenge in der Einrichtung. Beispielsweise ist es für die Einwirkung auf die Gelenke, die Füße und Arme ausreichend, eine Packung aus Filmen mit der Information der BAF oder auf einer Disk und für die Wiederherstellung der Funktion des Gehirns mindestens drei Disks oder Packungen mit der Information der BAF zu verwenden. Dadurch kann in diversen nach der Kompliziertheit geordneten Situationen eine hohe Wirkung stabil erzielt werden.

In der Einrichtung gemäß Anspruch 1 soll man für die Einwirkung auf breite Bereiche eine große Menge Disks und Platten, abgenommen von den Disks nach der Aufnahme der BAF auf diese, mit der Information verwenden. Als einzige Urquelle des biologisch aktiven Felds in dieser Einrichtung gilt die Information der BAF. Das begrenzt die Möglichkeiten der Einrichtung.

### Einrichtungsvariante 2

Zur Beseitigung dieses Mangels wurde der "Zellenförmige Feldharmonisator der Biosysteme" entwickelt, der eine Kombination in unterschiedlichen Verflechtungen aus symmetrischen richtigen Zellenformen als Zylinder, Prismen, liegend innerhalb der Zellen, miteinander kreuzenden und nichtkreuzenden Zylindern mit lichtabstrahlender Deckfläche darstellt und aus Disks oder Packungen oder Platten mit den Aufnahmen der BAF gemäß Anspruch 1 sowie aus Linsen, Konussen oder Pyramiden besteht, ausgeführt aus Glas, Kristall, Natur- oder Kunstmineralen, die einen allgemeinen Feldreflektor für jede der Zellen im Arbeitsbereich aus Spiegeln oder aus Folie besitzen.

Wie die Experimente von G.W. Worsunow zeigen, bestehen die Einrichtungen aus Zellen, die in Form von sechsflächigen symmetrischen Prismen oder Zylindern ausgeführt sind, innerhalb derer Zylinder mit kleinerer Nennweite in unterschiedlichen Varianten angeordnet sind, wobei jedoch die Lagesymmetrie eingehalten wird. Die Oberflächen dieser Zylinder weisen eine lichtreflektierende Abdeckung auf, wobei das Feld mit biologischer Aktivität eine bedingt wirkungsvolle Kapazität besitzt, die für die positive Einwirkung auf den Zustand des Organismus eines Menschen ausreicht (Man kann solche Einrichtungen mit Abmessungen von Nanometern bis zu Metern und mehr entwerfen). Es kann eine unendliche Menge von Ausführungsvarianten solcher Einrichtungen in Kombination mit Informationsträgern der BAF (Disks, Packungen aus Platten gemäß dem Anspruch 1, andere Informationsträger) mit Linsen, Pyramiden, Konussen aus Glas und aus diversen Kunst- und Naturmineralen geben, die die Felder fixieren und die Wirksamkeit der Verwendung dieser Einrichtungen vergrößern. Zu dieser Unmenge könnten auch gehören: angefangen von Juwelierwaren, Bekleidung, Teppichen, Mützen, hergestellt aus Fäden oder Schnur mit einer lichtabstrahlenden Abdeckung (zum Beispiel aus Alufolie) bis hin zu diversen Paneelen, Möbelbauelementen, Betten, Sesseln, unterschiedlichen Maschinen und Gebäuden. Besonders aktuell ist die Verwendung solcher Einrichtungen in Kindereinrichtungen und Heilanstalten. Die Entstehung der Ausstrahlungen in solchen Einrichtungen erfolgt auf Kosten der schon erwähnten "Wirkung der Hohlstrukturen" sowie der Wirkung der Form, beschrieben von dem Wissenschaftler G.I. Schipilow, Artikel 4 über die Wirkung der Form (www.fizvakum.narod.ru). Auch gibt es eine Information über die Formwirkung von anderen Wissenschaftlern aus Russland, wie A.E. Akimow (Akimow, A. E. "Physische Grundlagen der fundamentalen Begriffe der Lehre von Agni" in "Lebendige Ethik, Wissenschaft, Gesellschaft: Sammlung der Arbeiten des Akademiemitglieds der Russischen Akademie für Naturwissenschaften, des Direktors des Instituts für theoretische und angewandte Physik A. E. Akimow.", Pensa 2000, Seite 12 - 2).

Der Mechanismus der Einwirkung auf lebendige Organismen und Substanzen wirkt so, wie er in der Einrichtung gemäß Anspruch 1 beschrieben ist. Die Einrichtung sollte möglichst nahe am Körper des Benutzers auf eine solche Weise angelegt werden, dass die Felder in die Richtung auf den Körper ausstrahlen können. Die bedingte wirkungsvolle Kapazität der Felder, die von diesen Einrichtungen erzeugt werden, ist höher als in den Einrichtungen Nr. 1, da sich die Felder aller Quellen summieren. Die Effektivität der Einwirkung dieser Einrichtungen ist proportional der Anzahl der Zellen und dem Umfang, der von der ganzen Konstruktion belegt wird, sowie der Anzahl der verwendeten Einrichtungen gemäß Anspruch 1 und der die Felder fokussierenden Linsen, Konusse, Pyramiden aus Glas oder aus Mineralen.

### Einrichtungsvariante 3

Zur Beseitigung der Mängel, die den Einrichtungen gemäß den Varianten 1 und 2 eigen sind, wurde der "optische Feldharmonisator der Biosysteme" entwickelt, der ein oder mehrere Bündel aus optischen Fasern (hart oder flexibel) aufweist, wobei ein oder beide Enden der Bündel auf einer oder mehreren Einrichtungen gemäß der Variante 1 oder 2 oder auf einer beliebigen Kombination aus diesen Einrichtungen (oder Einrichtung) befestigt wird bzw. werden, wonach diese Einrichtungen (oder Einrichtung) zusammen mit den auf diesen befestigten Enden der optischen Faser in eine Folie eingewickelt oder in einer Kiste mit einer lichtreflektierenden Abdeckung untergebracht oder auf irgendeiner Oberfläche mit einer lichtreflektierenden Abdeckung befestigt werden. Dabei befindet sich die optische Faser entweder im freien Zustand oder wird auf irgendeiner Grundlage mit einer lichtreflektierenden Abdeckung befestigt. Die Verwendung der optischen Faser erlaubt, die Bündel auf einige Stücke zu verzweigen und gleichzeitig die Einwirkung einer Einrichtung gemäß der Varianten 1 oder 2 oder ihrer Kombination auf einige Biosysteme auszuführen. Das ist besonders aktuell für große Heilanstalten. Die Herstellung und Verwendung solcher Einrichtungen ist in einer unendlichen Menge von Varianten bei Teppichen, Decken, Elementen der Bekleidung, Sesseln, Betten, Räumen und Transportmitteln von Kinderwagen bis zu Flugzeugen, in Biotechnologien für die Übertragung der Felder der BAF in die notwendigen Bereiche usw. möglich. G.W. Worsunow verwendet für Experimente flexible optische Fasern mit einer Dicke von 0,15 - 1 mm, mit einer Länge von 0,1 - 30 m und Packungen aus Platten mit Aufnahmen der BAF, die eine Information von 96 Gbyte bis 3 Tbyte einspeichern. In diesen Einrichtungen widerspiegelt die reflektierende Abdeckung die Felder, die von den Filmen ausgestrahlt werden, und diese Felder gehen in die optische Faser ein. Wie die praktische Erfahrung der Verwendung solcher Einrichtungen zeigt, werden die Felder beim Durchgang der Felder von der Einrichtung gemäß Anspruch 1 über die Faser durch deren Seitenoberflächen ausgestrahlt. Aufgrund der Verwendung der optischen Faser nimmt die Fläche der ausstrahlenden Oberfläche sehr stark zu, und demzufolge erhöht sich die bedingte wirkungsvolle Kapazität der Einrichtung im Vergleich zu den Einrichtungsvarianten 1 und 2. Das ist durch Experimente mit Wasser, ausgeführt in der OOO-KTI, nachgewiesen worden. Die Einrichtungsvariante 1, in der OOO-KTI geprüft, enthält 96 Gbyte Information mit der BAF und besteht aus einer Disk und drei auf dieser fixierten Filmen mit Aufnahmen der BAF. Die Einrichtungen Nr. 2 und Nr. 3 bestehen auch aus einer Disk und drei Filmen mit 96 Gbyte derselben Information, aber in der Einrichtungsvariante 2 sind die Platten auf einer zellenförmigen Konstruktion fixiert, jedoch sind in der Einrichtungsvariante 3 auf dem Feld 300 optische Fasern mit einer Dicke von 0,15 mm und einer Länge von 200 - 250 mm fixiert. Die Reaktion des Wassers auf die Felder, die von der Einrichtung gemäß der Variante 1 ausgestrahlt werden, ist stärker als die Reaktion des Wassers auf die Felder der ersten Einrichtung. Auf diese Weise wurde nachgewiesen, dass die von der Oberfläche der Platten ausgestrahlten Felder, die die Information der BAF einspeichern, in die optischen Fasern eingehen und, über diese durchgehend, in den Raum ausgestrahlt werden.

Bei den Benutzern der Einrichtungen verstärken sich die Reaktionen des Organismus mit der Zunahme der Faser, die die Felder ausstrahlen.

Wenn die Fasern neben dem Körper des Benutzers mit Hilfe diverser Konstruktionen (flach und dreidimensional) untergebracht und auf der internen Oberfläche dieser Konstruktionen befestigt werden, kann eine komplexe Einwirkung auf mehrere Organismen und Systeme und, wenn nötig, auf den ganzen Organismus oder auf mehrere Menschen gleichzeitig ausgeführt werden, wobei nur eine Packung mit Platten mit der Information der BAF verwendet wird. Wenn sich die Anzahl der Informationen, die in der Einrichtung gemäß der Variante 1 eingespeichert ist, verändert, dabei diverse Einrichtungen gemäß der Variante 2 verändert und sich die summarische Fläche der Sektion und die Länge der optischen Faser verändert, über die das Feld verbreitet wird, können die von dieser Einrichtung geschaffenen Parameter des Felds geändert und dabei die höchste Wirkung erzielt werden. Wenn eine große Anzahl der optischen Fasern mit großer Länge verwendet wird, und diese auf den Boden der Wasserbecken gelegt oder an Floße in der nötigen Tiefe angehängt werden, kann wirkungsvoll auf den Zustand der Biosysteme in Gebieten von Flüssen, Teichen und Seen eingewirkt werden.

### Einrichtungsvariante 4

Der Mangel der Einrichtung gemäß der Variante 3 liegt in der Umständlichkeit der Organisation einer wirkungsvollen Einwirkung auf die Biosysteme in einem großen Raum. Zur Beseitigung dieses Mangels wurde der "Dynamische Harmonisator der Biosysteme" entwickelt, der einen drehbaren Gegenstand mit den auf diesem fixierten Einrichtungen gemäß den Varianten 1, 2 und 3 in beliebigen Kombinationen mit Berücksichtigung der realen technischen Möglichkeiten darstellt. Für Experimente wurden Haushaltslüfter verwendet. Ihre Schaufeln waren mit Alufolie bedeckt, und auf die Schaufeln waren Platten aus den Filmen mit Aufnahmen der BAF geklebt. Im Grunde ist es die drehbare Variante der Einrichtung gemäß der Variante 1. Wie die praktische Erfahrung zeigt, entsteht bei der Drehung der Schaufeln dieser Lüfter das Feld, das über eine höhere biologische Aktivität als das Feld verfügt, das von diesen Platten im unbeweglichen Zustand ausgestrahlt wird. Je höher die Geschwindigkeit der Drehung, desto höher ist die biologische Aktivität der Felder, die von dieser Einrichtung erzeugt wird. Diese Erscheinung wird dadurch erklärt, dass bei der Drehung der Quellen des biologisch aktiven Felds diese Gegenstände ein Torsionsfeld ausstrahlen. Bei der Drehung der Quellen des biologisch aktiven Felds entsteht ein zusätzliches Torsionsfeld, das sich mit dem von ihm ausgestrahlten Feld summiert. Diese Tatsache wurde durch Experimente in der OOO-KTI nachgewiesen. Die Wasserreaktion ist bei dem Drehlüfter mit der auf ihm fixierten Einrichtung gemäß der Variante 1 viel stärker als die von der unbeweglichen Einrichtung gemäß der Variante 1. Mit diesen Lüftern können zwei Aufgaben erfüllt werden, nämlich die Zirkulation von Luft und gleichzeitig die Schaffung eines Raums großen Umfangs des biologisch aktiven Felds für die Lösung der Gesundheitsprobleme der Menschen, die sich in dem Raum befinden, in dem der Lüfter steht. Die Verwendung solcher Einrichtungen ist in Lüftungssystemen der Reanimierabteilungen, in Hospizen, in Kindereinrichtungen, in Häusern für ältere Leute und in Hospitälern besonders aktuell. Nützlich ist auch die Verwendung in allen Wohn- und Industrieräumen und in allen Transportmitteln als Mittel zur Vorbeugung von Krankheiten, für die Sicherung eines hohen Tonus usw. Darüber hinaus ist es sehr nützlich, solche Einrichtungen in allen technischen Anlagen zu verwenden, in denen die Möglichkeit besteht, irgendwelche von diesen Einrichtungen gemäß den Varianten 1, 2, 3 auf sich bewegende Teile oder Baueinheiten dieser Einrichtungen zu befestigen. In erster Linie ist es notwendig, dies innerhalb der Reifen von Fahrzeugen und auf den Schaufeln von Lüftern, auf den Wellen von Wassergeneratoren und auf Scheiben von Schiffen auszuführen. Bei der Drehung der Räder, Schaufeln und Rotoren mit den auf ihnen fixierten Informationsträgern der BAF wird das biologisch aktive Feld in den Raum ständig ausgestrahlt, das positiv auf die Menschen, die sich innerhalb dieser Transportmittel befinden, und auf alle lebendige Organismen einwirkt, die sich im Bereich des Betriebs der Fahrzeuge und Generatoren befinden. Für das Experiment hat G.W. Worsunow auf die internen Oberflächen der Reifen seines Fahrzeugs Platten mit den Aufnahmen der BAF angeklebt. Bei Menschen mit Gesundheitsproblemen, die sich innerhalb des Fahrzeugs befinden, entstehen im Laufe der Bewegung des Fahrzeugs Reaktionen, die den Reaktionen auf die Einrichtungen gemäß den Varianten 1, 2 und 3 ähnlich sind. Wenn die Tatsache berücksichtigt wird, dass sich eine große Menge von Transportmitteln, Luft- und Wassergeneratoren und anderen diversen Mechanismen im bewegten Zustand befinden, kann man mit Sicherheit eine sehr hohe Wirkung in Bezug auf den Umsatz solcher Einrichtungen in der breiten Praxis für die Vorbeugung der Gesundheit, der sicheren Umwelt entlang der Transportstrecken und im Bereich von Windkraftwerken voraussagen. Die natürliche Fortbewegung von großen Luftmassen und von Wasser wird den Einwirkungsbereich dieser Einrichtungen auf viele Kilometer verbreiten, und es kann mit Sicherheit vorausgesagt werden, dass bei einem umfassenden Umsatz dieser Einrichtungen in der Praxis globale positive Umweltveränderungen erfolgen werden.

### Einrichtungsvariante 5

Diese Variante wird durch eine Kammer mit einer hohen Dichte der biologisch aktiven Felder für die Lösung von schwierigen Gesundheitsproblemen (für die Heilungsbeschleunigung von umfassenden Wunden, bei allen Formen der scharfen Insuffizienz, für die Hilfe für Kinder in Bezug auf die meisten Probleme einschließlich von Geburtsverletzungen, Onkologie usw.), für die Einwirkung auf unterschiedliche Substanzen zwecks Verbesserung der Verbrauchseigenschaften gebildet: Parfümerie, Wasser, Früchte, Gemüse, Lebensmittel im flüssigen und halbflüssigen Zustand oder mit Wassergehalt sowie Arbeitsgebiete der Biotechnologie zur Beschleunigung der Prozesse der Zellteilung und des Wachstums der Zellmasse, wobei die Kammer einen beliebigen geschlossenen Raum (Box, Raum, Gebäude) darstellt, dessen Innenflächen eine lichtreflektierende Abdeckung aufweisen und innerhalb derer eine beliebige der vorgenannten Einrichtungen oder ihrer Kombination liegt. Die Einwirkung auf den Organismus und die Substanzen in solchen Kameras haben einen stärkeren Charakter und eine höhere Wirkung als die Einwirkung der Einrichtungen gemäß den Varianten 1 - 4. Dies wird damit erklärt, dass die Felder, die von den Einrichtungen gemäß den Varianten 1 - 4 ausgestrahlt werden, nicht über die Grenzen des Raums hinaus gehen, der mit der lichtreflektierenden Abdeckung beschränkt ist. Die in solchen Einrichtungen drei oder mehrere Tage aufbewahrte Parfümerie übt auf die Haut eine viel stärkere erfrischende, verjüngende Einwirkung aus als die Ausgangsparfümerie. Es ist sehr aktuell, diese Einrichtung als Verpackung für Parfümerieprodukte zu verwenden. Wasser, Früchte, flüssige Lebensmittel erwerben einen angenehmeren Geschmack nach der Aufbewahrung im Kühlschrank, der die Einrichtung bildete (innerhalb des Schranks liegt der Harmonisator gemäß der Variante 3, und die Innenflächen sind mit einer Folie bedeckt; eine Kartoffel wird schneller dunkel, wenn sie in Teile zerschnitten ist.) Theoretisch soll die Aufbewahrung von Arzneimitteln in einer solchen Kammer eine Verstärkung der heilenden Wirkung ergeben. Eine GDV-Kamera registriert die Reaktionen der Zahnpasta und Parfümcreme nach ihrer Aufbewahrung im Kühlschrank im Lauf von bis zu fünf Tagen.

### Einrichtungsvariante 6

Die Enden der Bündel der optischen Faser werden innerhalb der Einrichtung gemäß der Variante 5 fixiert. Eine derartige Einrichtung erlaubt die Verwendung einer großen Menge von Feldquellen und erlaubt weiterhin, die Reichweite der Aufgaben zu erhöhen, die mit maximaler Wirkung gelöst werden.

### Einrichtungsvariante 7

Die Einrichtung dieses leuchtenden Harmonisators für Biosysteme weist ein Bündel oder einige Bündel aus optischen Fasern auf, auf deren Eingänge die BAF mittels der Linse eines Projektors übertragen wird. Die Faserbündel können in einer Ebene und in verschiedenen dreidimensionalen Strukturen in Verbindung mit dem Reflektor des Felds in der Arbeitszone angeordnet sein. Beim Durchgang der Darstellung der BAF durch die Fasern, wie in der Einrichtung gemäß der Variante 3, wird das biologisch aktive Feld durch ihre Seitenoberflächen ausgestrahlt. Die Bündel der optischen Fasern können verzweigt werden, und von einem Projektor aus kann die Einwirkung auf einige Menschen ausgeübt werden, oder die Felder der BAF können in einige Arbeitsbereiche übergeben werden. Bei der Verwendung der optischen Faser mit einer guten Lumineszenz können solche Einrichtungen gleichzeitig als Beleuchtungssystem verwendet werden. Das ist besonders aktuell für die Verwendung in Krankenhäusern oder bei gesundheitsschädlichen Produktionen usw.

### Einrichtungsvariante 8

Den Harmonisator der Biosysteme mit Laser stellt die Einrichtung gemäß der Variante 3 dar. Auf den Eingang in die optischen Fasern wird aber der Strahl des Lasers oder einiger Laser, moduliert mit der Videoaufnahme der BAF, gegeben. Alternativ werden in einem Korb mit den Einrichtungen gemäß den Varianten 1 und 2 zusätzlich ein oder mehrere Laser auf eine solche Weise untergebracht, dass dessen Strahl bzw. deren Strahlen in die optischen Fasern hineingeht bzw. hineingehen. In dieser Einrichtung erfasst der Strahl des Lasers die Information, die von den Feldern der Einrichtungen gemäß den Varianten 1 und 2 ausgestrahlt werden und trägt sie zur optischen Faser. Dabei wird auch wie in der Einrichtung gemäß der Variante 3 durch die Seitenoberflächen das biologisch aktive Feld ausgestrahlt, das im Organismus unterschiedliche positive Prozesse einleitet. Die Ausstrahlung des elektromagnetischen Felds gilt als eine der physischen Eigenschaften des Lasers. Der Doktor für Biologie P.P. Gariaev verwendet in seinen Experimenten für die Wellengenetik die Eigenschaft des Laserstrahls, die Information zu erfassen und diese in den Raum auszustrahlen. In der optischen Faser wird der Strahl des Lasers ohne Verlust auf sehr große Dimensionen übertragen, so dass die biologisch aktiven Felder mit großer bedingter Leistungskapazität in sehr große Räume geschafft und diese auf Kosten der Verzweigung auf eine große Anzahl der Biosystemen auf große Dimensionen übertragen werden.

Die Einrichtung für die Modulation des Laserstrahls ist wegen ihrer Kompliziertheit noch nicht hergestellt, aber in der Praxis der Verwendung der BAF gab es einen Fall, in dem bei einem Benutzer das Fernsehgerät gestört war und dem Benutzer die Wiederaufnahme der BAF nur auf einem DVD-Player möglich war. Dabei gab es bei dem Benutzer positive Reaktionen. Das bedeutet, dass das vom Laser des DVD-Players ausgestrahlte Feld bei der Wiederaufnahme der BAF positiv auf den Organismus eines Menschen wirkte (als Benutzer trat ein älterer Mann mit einem schlechten Gedächtnis auf). Diese praktische Erfahrung mittels der Experimente von P.P. Gariaev gilt als Grundlage für die Entwicklung dieser Einrichtung.

### Einrichtungsvariante 9

Im Harmonisator der Biosysteme mit einem offenen Laser wird der Strahl des Lasers oder der Laser, der bzw. die in einer Kammer mit einer lichtstrahlenden Abdeckung der internen Oberflächen angeordnet ist bzw. sind, durch eine Öffnung an der Kammerwand nicht in die optische Faser sondern in den Raum hinter der Kammer in den Arbeitsbereich gerichtet, d. h. zum Biosystem, das die Einwirkung braucht. Das Feld in der Kammer wird mit dem Strahl des Lasers (der Laser) erfasst und in den Raum ausgestrahlt, wobei es auf die neben dem Strahl liegenden Biosysteme einwirkt.

Diese Variante des Harmonisators ist einfach und bequem und erlaubt, die Einwirkung auf große Dimensionen und in großen Umfängen des Raums auszuüben. Sie ist effektiv für die Beseitigung von Krankheiten, und eine Verwendung in den Biotechnologien und Technologien für die Umwelt ist möglich.

Diese technische Lösung ist in der Produktion verwendbar, da in der Beschreibung zum Antrag und in der Bezeichnung des Modells ihre Bestimmung angegeben ist. Sie kann produktionsmäßig hergestellt werden und für die Herstellung des Feldharmonisators der Biosysteme und Substanzen auf Disks für die Schaffung der Felder verwendet werden, die positiv auf lebendige Organismen einwirken. Sie ist ausführbar und produzierbar, und die Eigenschaften der Einrichtung erlauben, das vorgegebene technisch erhebliche Ergebnis zu erhalten.
Die Erfindung in derjenigen Form, wie sie in jedem der beigefügten Ansprüche charakterisiert ist, kann mit Hilfe der für den bekannten Prototyp beschriebenen Mittel und Methoden verwirklicht werden. Folglich entspricht die angegebene technische Lösung dem Niveau der Patentfähigkeit "Produktionsanwendbarkeit".

Die Wirkung der Arbeit aller angebotenen Einrichtungen wird sehr leicht in der Praxis bestätigt, und Interessenten können ihre Prüfungen sehr leicht vornehmen.

## Patentansprüche

1. Feldharmonisator für Biosysteme und Substanzen mit Disks für die Schaffung von Feldern, die positiv auf lebendige Organismen einwirken, einschließlich des Organismus eines Menschen und Substanzen, wobei der Feldharmonisator eine Disk, auf der 23.5 und mehr Gbyte von biologisch aktiven Filmen (kurz BAF) von G.W. Worsunow gespeichert sind, oder eine Disk und auf dieser fixierte Platten mit Filmen, die von den Disks nach der Aufnahme der BAF auf diese Disks abgenommen sind, oder eine bestimmte Anzahl solcher Platten, die in Packungen gelegt sind, oder andere Informationsträger enthält, die eine Information mit einer Qualität und Quantität tragen, die für die Anregung von positiven Änderungen in Biosystemen ausreichend sind, wobei die Informationsträger auf einer beliebigen Oberfläche mit einer lichtstrahlenden Abdeckung aus Alufolie, Spiegeln oder aus poliertem Metall usw. für die Reflektion des Felds zum Biosystem angeordnet sind.

2. Feldharmonisator für Biosysteme und Substanzen nach Anspruch 1,
**dadurch gekennzeichnet,**
**dass** er Konstruktionen einschließt, die aus Zellen und Formen bestehen, die in großer Menge vorhanden sein können, die über allgemeine Merkmale verfügen, die die Form der äußeren Kontur der Zellen in Form von richtigen vielflächigen Prismen oder Zylindern und die symmetrische Lage innerhalb der Zellen in ihrem Verhältnis zur Achse betreffen, wobei die Zylinder und Prismen eine geringe Größe aufweisen und sich teilweise miteinander kreuzen, eine lichtabstrahlende Abdeckung oder polierte Oberflächen mit liegenden, von der Außenseite der Felder in Bezug auf das Biosystem der Einrichtungen (gemäß der Einrichtungsvariante 1) und innerhalb der Zellen liegenden Linsen, Konussen oder Pyramiden aus Glas, Kristall oder aus Mineralen aufweisen, die mit dem scharfen Ende zum Biosystem gerichtet sind, und dass er allgemeine für die ganze Einrichtung oder für jede Zelle vorgesehene Reflektoren des Felds aus Alufolie oder aus Spiegeln enthält, die die Felder zum Biosystem ausstrahlen.

3. Feldharmonisator für Biosysteme und Substanzen nach Anspruch 1 oder 2, **dadurch gekennzeichnet, dass**
- auf der Oberfläche dieser Einrichtung oder Einrichtungen ein oder mehrere Bündel aus optischen Fasern mit einem Ende oder beiden Enden befestigt sind,
- der Harmonisator nach Anspruch 1 oder 2 oder einer Kombination daraus zusammen mit den auf den Einrichtungsoberflächen befestigten optischen Faserenden entweder auf der Oberfläche mit einer reflektierenden Abdeckung befestigt oder in einer Aluminiumfolie eingewickelt oder innerhalb einer Box angeordnet werden, deren Innenflächen aus Spiegeln bestehen oder eine andere lichtreflektierende Abdeckung aufweisen,
- die optischen Fasern außerhalb der Box und des Spiegelbelags angeordnet sind
- oder die optischen Fasern in freiem Zustand angeordnet sind und für den Transport des Felds zu einem Biosystem oder einer Substanz verwendet werden,
- oder die optischen Fasern in mehrere Bündel verzweigt werden und das Feld in mehrere biologische Systeme oder Substanzen gleichzeitig transportieren,
- die optischen Fasern auf der Spiegelfläche oder einer anderen Fläche mit einer lichtreflektierenden Abdeckung befestigt sind, wobei die Fläche Strahlen aussendet,
- oder die optischen Fasern gleichmäßig auf einem Rahmen aufgewickelt sind mit einer folgenden Befestigung auf einer Schicht eines optische Fasern aufweisenden Reflektors, der aus Spiegeln oder Aluminiumfolie besteht, die das Feld in einem durch die Fasern begrenzten Innenraum reflektieren, in dem ein Biosystem in Erwartung des Felds angeordnet ist.

4. Feldharmonisator für Biosysteme und Substanzen nach einem der Ansprüche 1 bis 3,
**dadurch gekennzeichnet,**
**dass** er auf der Oberfläche von beliebigen rotierenden Teilen oder von deren Innenhohlräumen angeordnet ist, beispielsweise von Flügeln, Rotoren von Überkopflüftern, Luft- und Wassergeneratoren, Schiffspropellern, Fahrzeugrädern und Reifen von Autos und anderen Fahrzeugen, wobei die rotierenden Elemente dieser Teile für die Erzeugung und Aussendung in den Raum von biologisch aktiven Feldern besonders ausgebildet sind, die die biologischen Systeme und Substanzen positiv beeinflussen und
**dass** die befestigte Einrichtung nach Anspruch 1, 2 oder 3 in verschiedenen Varianten für entsprechende Aufgaben und technische Möglichkeiten der Befestigung und Betriebsweise vorgesehen ist.

5. Feldharmonisator für Biosysteme und Substanzen nach einem der Ansprüche 1 bis 4 für ein breites Verwendungsspektrum, und zwar für alle möglichen Formen und Abmessungen, für die Aufbewahrung von Parfümerieprodukten, Wasser, Lebensmitteln, Alkoholprodukten, Arzneimitteln bis hin zu Kammern und Räumen für die Lösung von Gesundheitsproblemen und zu Biotechnologien,
**dadurch gekennzeichnet,**
**dass** er einen beliebigen Raum aufweist, der durch Oberflächen mit einer lichtreflektierenden Abdeckung versehen ist und in dem die Einrichtung nach den Ansprüchen 1, 2, 3 oder 4 in Kombinationen liegen, die den mit Hilfe dieser Einrichtung zu lösenden Aufgaben entsprechen.

6. Feldharmonisatorfür Biosysteme und Substanzen nach Anspruch 3,
**dadurch gekennzeichnet,**
**dass** die Enden der Bündel der optischen Fasern gemäß dem Anspruch 5 befestigt werden.

7. Feldharmonisator für Biosysteme und Substanzen nach Anspruch 3,
**dadurch gekennzeichnet,**
**dass** er in Verbindung mit Licht in sich die Funktionen einer Quelle des biologisch aktiven Felds und eines Beleuchtungssystems vereinigt und
**dass** auf den Eingang in die optische Faser das Video der Darstellung der BAF mit der Seitenluminiszenz durch das Objektiv eines Projektors übertragen wird.

8. Feldharmonisator für Biosysteme und Substanzen nach Anspruch 3 mit einem Laser,
**dadurch gekennzeichnet,**
**dass** auf den Eingang in die optische Faser der Strahl eines Lasers oder die Strahlen von Lasern gegeben wird bzw. werden, wobei die Strahlen ein moduliertes Video mit der Darstellung der BAF enthalten oder die Strahlen vom Laser nicht moduliert sind oder der Laser innerhalb der Einrichtung nach Anspruch 5 oder 3 liegt.

9. Feldharmonisator für Biosysteme und Substanzen nach Anspruch 8 mit einem offenen Laser,
**dadurch gekennzeichnet,**
**dass** der Strahl des Lasers oder die Strahlen der Laser, die in einer Box (Kiste) liegen, sich durch eine Öffnung an der Wand der Kammer zum Biosystem bewegt bzw. bewegen, das die Einwirkung braucht.
